# EUROPEAN PATENT SPECIFICATION

(11) **EP 0 506 240 B1**
(45) Date of publication and mention of the grant of the patent: **07.02.1996**
(21) Application number: 92301825.3
(22) Date of filing: 04.03.1992
(51) Int. Cl.: C07C 51/44, C07C 51/12

(54) **Process for the recovery of acetic acid from compositions comprising acetic acid and water**
Verfahren zur Wiedergewinnung von Essigsäure aus Essigsäure und Wasser enthaltenden Zusammensetzungen
Procédé pour la récupération de l'acide acétique à partir des compositions contenant de l'acide acétique et de l'eau

(30) Priority: 25.03.1991 GB 9106298
(43) Date of publication of application: 30.09.1992
(73) Proprietor: BP Chemicals Limited, London EC2M 7BA (GB)
(72) Inventor: Watson, Derrick John, Hedon, Hull HU12 8DS (GB)
(74) Representative: Perkins, Nicholas David

(56) References cited:
- EP-A- 0 055 618
- EP-A- 0 134 650
- EP-A- 0 144 935
- EP-A- 0 161 874
- EP-A- 0 250 189
- DE-A- 2 119 744
- DE-B- 2 223 541
- GB-A- 2 146 637
- MONATSHEFTE F R CHEMIE, vol. 99, no. 3, 1968, pages 913 - 917 T. SCH NFELD ET AL. 'Zur Zusammensetzung des Dampfes bei der Destillation von Essig s ure-Wasser-Calciumchlorid-Gemischen'
- TAIWAN SCIENCE, Vol 23, No 3-4, 1969 , pages 43-45 C.Y.FENG ET AL

## Description

This invention relates to a separation process and in particular to a process for recovering acetic acid from a composition comprising acetic acid and water.

Acetic acid may be produced by a variety of processes, for example oxidation of hydrocarbons and carbonylation of methanol, dimethyl ether, methyl iodide and/or methyl acetate. It is often the case that the acetic acid product from such processes contains water from which it is desirable to separate the acetic acid.

Recovery of acetic acid from product compositions comprising water and acetic acid is known. Such known processes may comprise partitioning the composition into vapour and liquid phases which are then separated, at least a portion of the acetic acid from the composition being recovered in the vapour phase. However, when a composition comprising acetic acid and water is partitioned into vapour and liquid phases, some water will be present in the vapour phase with the portion of vapourised acetic acid. The water in the vapour phase requires further separation steps or processes.

The problem to be solved therefore, is to provide an improved process for the recovery of acetic acid from a composition comprising acetic acid and water.

European patent application number EP 0134650 describes a process for separating acetic acid from a moderately dilute aqueous solution which comprises subjecting the acid solution to an extraction treatment in the liquid phase with a high boiling solvent for acetic acid to extract the acid from the aqeuous solution.

US 3791935 and GB 1294432 relate to distillative processes for the purification of a carboxylic acid stream containing a halogen or a halogen-containing contaminant.

European patent application number EP 0161874 A1 describes a process for the production of a carboxylic acid by carbonylation of an alcohol, exemplified by the carbonylation of methanol to acetic acid. EP 0161874 recognises that water is an undesirable component of the crude acetic acid and that the more water there is in this product steam, the greater will be the operating costs and required capital investment in the product recovery-purification system. EP 0161874 A1 seeks to reduce the water content of the crude acetic acid by decreasing the water concentration in the carbonylation reaction medium. According to EP 0161874 A1 a metal iodide salt or an iodide salt of an organic cation is used to stabilize the catalyst and lithium iodide is said to be preferred. EP 0161874 A1 does not describe the use of selected iodides to suppress the volatility of water relative to acetic acid.

UK published patent application GB-A-2146637 describes production of carboxylic acids or esters by liquid phase carbonylation of an alcohol in the presence of a rhodium catalyst an alkyl iodide or bromide and water in which an iodide salt is added to maintain the iodide ion concentration in the carbonylation reaction solution at 0.3 mol/l or higher. GB-A-2146637 does not describe the use of selected iodides to suppress the volatility of water relative to acetic acid.

The use of lithium iodide as a carbonylation promoter is described in EP-A-0144935, EP-A-0144936, EP 0250189 and US-A-5003104. These publications do not describe its use to suppress the volatility of water relative to acetic acid.

US patent US-A-4733006 describes a rhodium-catalysed carbonylation process in which an alkali metal compound or a germanium (IV) compound is used as a catalyst stabilizer to prevent precipitation of rhodium catalyst during separation of carbonylation products under carbon monoxide deficient conditions.

The effects of various salts on the relative volatility of acetic acid with respect to water is described by C Y Feng et al in Taiwan K'ehs'ue (Taiwan Science) vol 23 no. 3-4 p43-5 (1969). Experiments using K₂SO₄, Na₂SO₄, KCl, NaCl, CaCl₂ and sodium acetate are described. There is no description of the effect of iodides.

Schonfeld et al in Monatshefte für Chemie 99 (1968) 913-917 describe distillation of acetic acid/water/calcium chloride mixtures. There is no description of the use of iodides.

Thus, according to the present invention there is provided a process for recovering acetic acid from a composition comprising acetic acid and water by the steps of (a) subjecting the composition to conditions of temperature and pressure to produce a vapour phase comprising at least a portion of the acetic acid in the composition and a liquid phase comprising the remaining portion of the acetic acid in the composition and (b) separating the vapour and liquid phases, characterised in that the liquid phase further comprises, one or more iodides of the elements of Group IA or Group IIA of the Periodic Table of the Elements or of hydrogen to suppress the volatility of the water relative to the acetic acid.

The present invention solves the problem defined above by the use of one or more iodides of Group IA or Group IIA or of hydrogen to suppress the volatility of water relative to acetic acid in a process for the recovery of acetic acid from a composition comprising acetic acid and water, which process comprises partitioning the composition into a vapour phase comprising at least a portion of the acetic acid in the composition and a liquid phase comprising the remaining portion of the acetic acid in the composition and separating the phases. Since residual water present in the recovered acetic acid can impose a significant energy burden for subsequent further separation of the water and acetic acid and/or limit throughput of the process, a reduction in the water content of the separated vapour phase can have a significant benefit for an acetic acid production process.

The conditions of temperature and pressure will depend upon the presence or absence of other compounds in the composition as well as the relative amounts of acetic acid and water in the composition. The conditions are selected so that the acetic acid is partitioned between the vapour and liquid phases. The temperature is above the freezing point of the liquid phase and not so high that all the composition is vapourised or that undesired breakdown of the composition and/or its components takes place. Suitably the temperature is in the range 50°C to 300°C preferably in the range 50°C to 200°C. The pressure may be subatmospheric, atmospheric or superatmospheric and is selected according to the temperature and composition to provide the desired partitioning of acetic acid between the vapour and liquid phases. Suitably the pressure is in the range of 1 x 10⁴ to 5 x 10⁶ Pa (0.1 bara to 50 bara), preferably 1 x 10⁴ to 1 x 10⁶ Pa (0.1 bara to 10 bara).

The iodide may be added directly to the liquid phase and/or added indirectly by adding it to the composition prior to partitioning into vapour and liquid phases. The iodide may be added to the composition or liquid phase in a form which is converted into the iodide in situ in the composition or the liquid phase for example as the corresponding acetate salt which is converted to the required iodide by an iodide component present in the composition or liquid phase.

The iodide may be added as a solid but is preferably added as a solution in a solvent compatible with the liquid phase, for example water and/or acetic acid or any other suitable solvent.

For the avoidance of doubt, the elements of Group IA of the Periodic Table of the Elements are lithium, sodium, potassium, rubidium, cesium and francium. The elements of Group IIA of the Periodic Table of the Elements are beryllium, magnesium, calcium, strontium, barium and radium.

Preferably the iodide is hydrogen iodide, calcium iodide, lithium iodide, potassium iodide and/or sodium iodide. More preferably, the iodide is hydrogen iodide, calcium iodide, lithium iodide and/or potassium iodide. A most preferred iodide is hydrogen iodide, calcium iodide and/or lithium iodide. Without wishing to be bound by any theory it is believed that iodides having cations with a high charge density are preferred in the process of the present invention.

When the iodide is hydrogen iodide sufficient water must be present to reduce the volatility of the hydrogen iodide so that the liquid phase comprises an effective amount of hydrogen iodide. This may be typically about 5 weight % water.

The amount of iodide added to the liquid phase should be an effective amount, sufficient to achieve the desired reduction in volatility of water relative to acetic acid and up to the limit of solubility of the iodide in the liquid phase. Suitable concentrations of iodide in the liquid phase are 0.1 to 50% by weight, subject to the limit of solubility of the iodide in the liquid phase.

Other compounds may be present in the composition with the acetic acid and water. These compounds may also be partitioned between the vapour and liquid phases. Thus, for example, if the composition comprises the carbonylation reaction composition of a process for the production of acetic acid by the carbonylation of methanol, methyl iodide, methyl acetate and/or dimethyl ether in the presence of at least a finite amount of water, in addition to the acetic acid and water, such composition may comprise methyl iodide and hydrogen iodide carbonylation promoters, involatile carbonylation catalyst components such as a Group VIII metal complex (for example a rhodium compound) and unreacted carbonylation reactant. When such a composition is partitioned into vapour and liquid phases, the low boiling components, such as methyl iodide, some hydrogen iodide and unreacted carbonylation reactants will predominantly partition into the vapour phase with at least a portion of the acetic acid and some of the water and the involatile carbonylation catalyst components will partition into the liquid phase with the remaining portions of the acetic acid and water. The addition of an iodide to the liquid phase according to the process of the present invention, will therefore reduce the amount of water partitioned into the vapour phase with the acetic acid so that the vapour phase will contain less water with respect to acetic acid than without the addition of the iodide. The carbonylation reaction may comprise reacting methanol, methyl iodide, methyl acetate and/or dimethyl ether with carbon monoxide in the liquid phase in a reaction zone in the presence of a carbonylation catalyst and at least a finite amount of water at a temperature of 50 to 400°C and a carbon monoxide pressure of 7 x 10³ to 1 x 10⁸ pag (1 to 15000 psig) to produce acetic acid and withdrawing liquid reaction composition from the reaction zone.

The mole ratio of water : acetic acid in the composition comprising water and acetic acid suitably may be about from 0.001:0.999 to 0.9:0.1.

The process of the present invention may be performed as a batch or a continuous process, preferably as a continuous process.

The process of the present invention may be performed by subjecting a liquid composition comprising acetic acid and water to an elevated temperature and pressure and then introducing it with the iodide (together or separately) into a flash zone from which the vapour phase and liquid phase are withdrawn separately. The flash zone is a zone at a pressure below the elevated pressure and with or without means for providing heat to the composition and vapour and liquid phases.

The use of a flash zone is particularly applicable when the composition is the liquid reaction composition of a carbonylation process for the production of acetic acid since such carbonylation process will be suitably performed at elevated temperature and pressure. The use of a flash zone to recover acetic acid from a liquid carbonylation product is described in US patent number 3,845,121.

Thus, according to one embodiment of the present invention there is provided the use of one or more iodides of Group IA or Group IIA of the Periodic Table of the Elements or of hydrogen for suppressing the volatility of water relative to acetic acid in a process for the production of acetic acid which comprises:
a) reacting methanol, methyl iodide, methyl acetate and/or dimethyl ether with carbon monoxide in the liquid phase in a reaction zone in the presence of a carbonylation catalyst and at least a finite amount of water at a temperature of 50 to 400°C and a carbon monoxide pressure of 7 x 10³ to 1 x 10⁸ Pag (1 to 15000 psig) to produce acetic acid,
b) withdrawing liquid reaction composition from the reaction zone,
c) introducing the liquid reaction composition into a flash zone at a pressure below that of the reaction zone with or without additional heating to produce a liquid phase and a vapour phase comprising a portion of the acetic acid carbonylation product and water,
d) introducing to the flash zone together with or separately from the liquid reaction composition one or more iodides of the elements of Group IA or Group IIA of the Periodic Table of the Elements or of hydrogen, and
e) separately removing from the flash zone the vapour phase and the liquid phase.

The vapour phase may be subjected to further separation processes to recover acetic acid product free of unconverted carbonylation reactant and promoters. These other compounds may be recycled to the carbonylation reaction zone. The liquid phase which comprises involatile carbonylation catalyst components, may be recycled directly to the carbonylation reaction zone. A suitable carbonylation catalyst comprises a Group VIII metal compound such as a compound of rhodium, optionally together with a suitable promoter such as alkyl iodide or bromide, for example methyl iodide and optionally an additional co-promoter such as lithium iodide or certain classes of imidazolium iodides, alkyl substituted pyridinium iodides and hydroxypyridinium iodides. Suitable carbonylation catalysts are described in our European patent publication EP 0391680 A1, and in US patent number 4,690,912.

In this embodiment of the present invention the involatile iodide will be removed from the flash zone in the liquid phase. If the liquid phase is recycled to the reaction zone the iodide compound will also be recycled to the reaction zone and at least a portion of the recycled iodide will be withdrawn with the liquid reaction composition. Thus, following an initial addition of iodide, the iodide will thereafter pass to the flash zone as part of the liquid carbonylation reaction composition. Additional amounts of iodide may be required from time to time or on a continuous basis to make good any losses from the system.

In this embodiment of the present invention, the presence of the iodide reduces the amount of water recovered with the acetic acid in the vapour phase from the flash zone. This will reduce the load on subsequent separation processes where acetic acid is recovered free of the other components in the vapour phase, including water.

The process of the present invention also may be performed by introducing the composition comprising acetic acid and water into a fractional distillation zone comprising one or more separation stages. The iodides may be introduced into the distillation zone together with, or separately from, the composition. Lower boiling components of the composition are recovered as a heads product stream and higher boiling components are recovered as a base product stream. At least a portion of the acetic acid together with part of the water from the composition may be recovered at a suitable point above the base of the distillation zone optionally as a heads product. The amount of water present with the acetic acid may be reduced by the addition of the iodide. This may assist in any further separation process to which the acetic acid-containing product stream may be subjected. A fractional distillation zone may be used for recovery of acetic acid when the composition comprising acetic acid and water is the liquid reaction composition of a carbonylation process for the production of acetic acid provided that the components in the composition such as the carbonylation catalyst are suitably stable in the distillation zone.

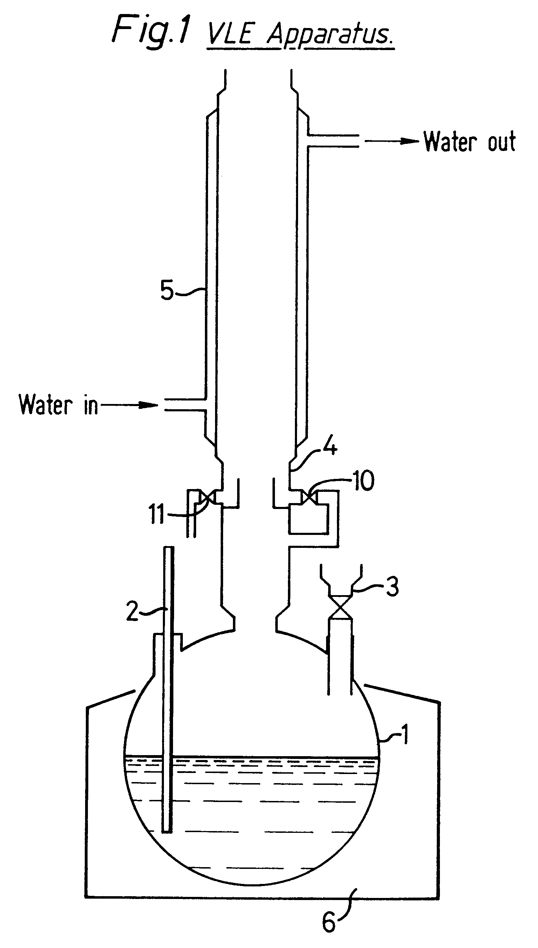

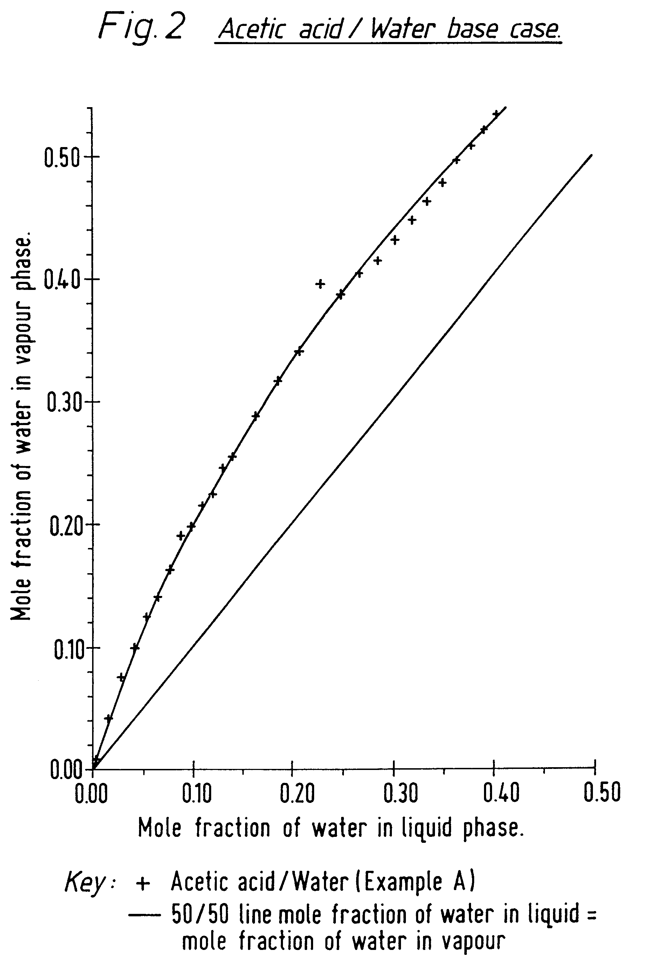

The invention will now be illustated by reference to the following Examples with reference to the accompanying drawings in which Figure 1 represents in schematic form the apparatus used to collect vapour/liquid equilibrium (VLE) data for various compositions comprising acetic acid and water and Figures 2 to 10 represent in graph form vapour/liquid equilibrium graphs for acetic acid-water compositions according to and not according to the present invention.

The apparatus used to measure the effect of various iodides on the relative volatilities of water and acetic acid is shown schematically in Figure 1.

Referring to Figure 1, a 500ml 3 necked round bottomed flask (1) was provided with a thermometer (2) and an addition port (3). The centre neck was provided with a chimney tray (4) surmounted by a water-cooled condenser (5). The chimney tray was provided with a first tap (10) to allow return of collected condensation to the flask and a second tap (11) for collecting samples. The flask was provided with a magnetic stirrer (not shown) and a heating mantle (6).

### Example A (Comparative Example)

500g of glacial acetic acid was charged to the flask, tap (10) was opened and tap (11) closed. The stirrer was started and the composition brought to gentle reflux using the heating mantle. The boiling point of the composition was recorded using thermometer (2). After about 30 minutes, tap (10) was closed and a 2g sample was collected through tap (11). The sample was analysed for water content by Karl Fischer analysis. An amount of water (2g) equivalent to the weight of sample removed was added to the flask, tap (10) was opened and tap (11) closed. The composition was allowed to reflux for about 30 minutes before the sampling procedure was repeated and a further replacement aliquot of water was added. This procedure was repeated until a total of 30g of water had been added. The procedure was continued taking 5g samples to be replaced by 5g aliquots of water until a further 50g of water had been added.

The water content of the composition was calculated after each addition and checked by analysis approximately every nine additions. In this way the composition of the vapour phase from an acetic acid-water composition with increasing amounts of water was determined. The results are shown in graph form in Figure 2 as the mole fraction of water in the vapour and liquid phases.

This is not an example according to the present invention as it does not contain an added iodide.

### Example B (Comparative Example)

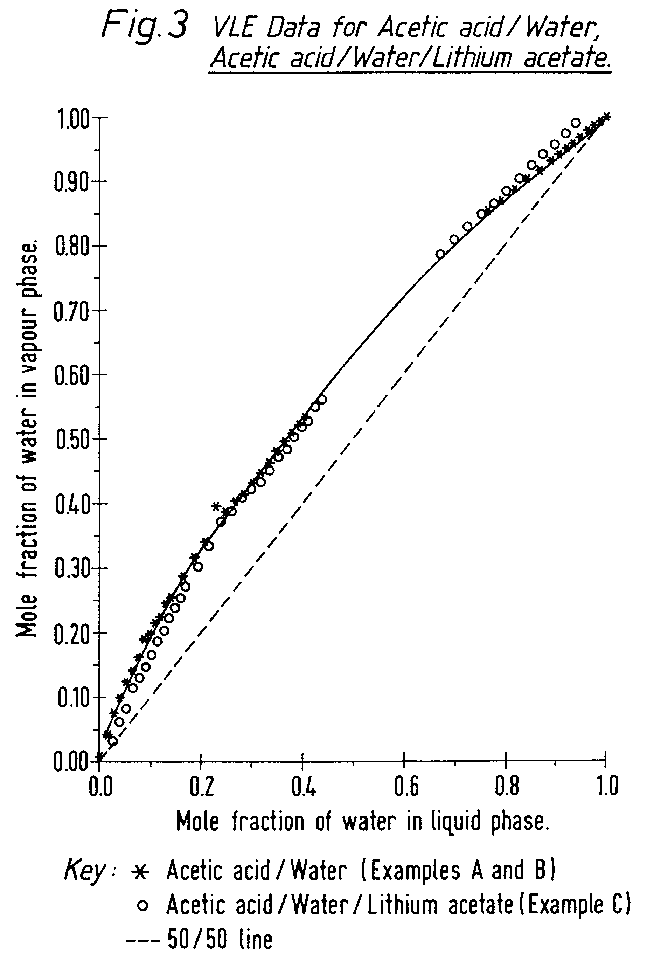

To complete the data for water-acetic acid compositions at high water concentrations Example A was repeated but using an initial change of 498g water and 2g glacial acetic acid instead of an initial charge of acetic acid and with the addition of aliquots of acetic acid to replace the samples taken. The water content was determined in this case by gas chromatography. The sampling and addition was repeated until 100g of acid had been added in 5g steps followed by 50g in 10g steps and 125g in 25g steps. The results are also shown in Figure 3.

From the graph it will be seen that there is a higher proportion of water in the vapour phase than in the liquid phase.

### Example C (Comparative Example)

Examples A and B were repeated using 13100 ppm lithium (0.94 moles) added to the starting composition as lithium acetate. Since this material was used as a hydrated salt, the water of crystallisation was removed by adding an equivalent amount of acetic anhydride to the composition and the water content was measured by Karl-Fischer. The results are shown in Figure 3.

This is not an example according to the present invention because an iodide was not used.

### Examples D and E (Comparative Examples)

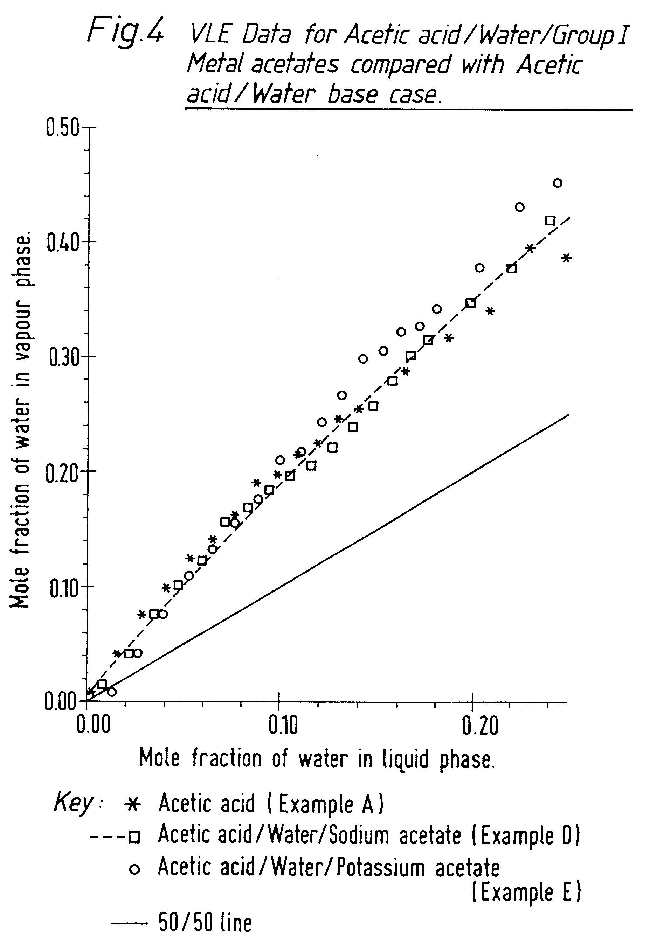

Example A was repeated using sodium acetate (0.48 moles) and potassium acetate (0.48 moles). The results are shown in Figure 4.

These are not examples according to the present invention because an iodide was not used.

### Example 1

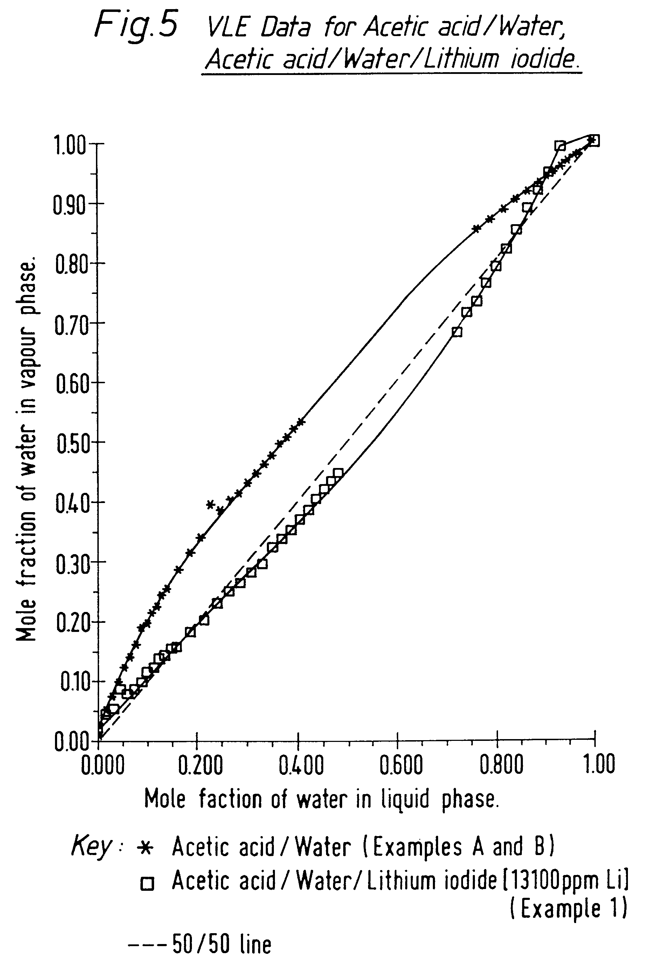

Examples A and B were repeated using 13100 ppm lithium added as lithium iodide (25.0 weight %) to the starting composition. The results are shown in Figure 5 and show a significant reduction of the volatility of water relative to acetic acid over a wide range of water concentrations.

### Examples 2 and 3

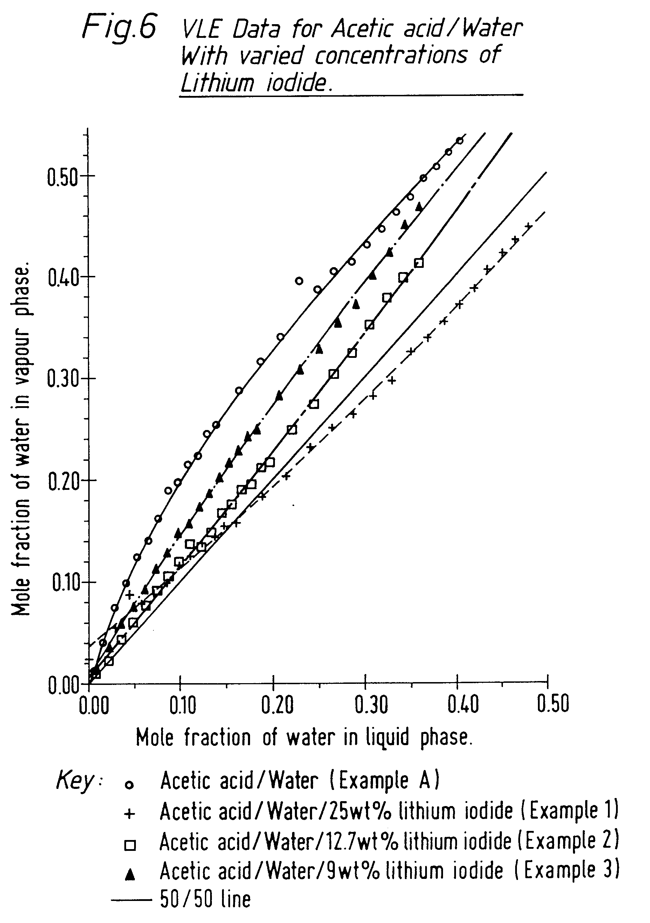

Example 1 was repeated using two different concentrations of lithium iodide added to the initial acetic acid-water composition (12.7 and 9 weight % respectively) and the results are shown in Figure 6. From the graph it is clear that the suppression of water volatility relative to acetic acid increases as the concentration of lithium iodide is increased.

### Examples 4 and 5

Example 2 was repeated using equimolar amounts of sodium and potassium iodides (14.17 weight % and 15.70 weight % respectively).

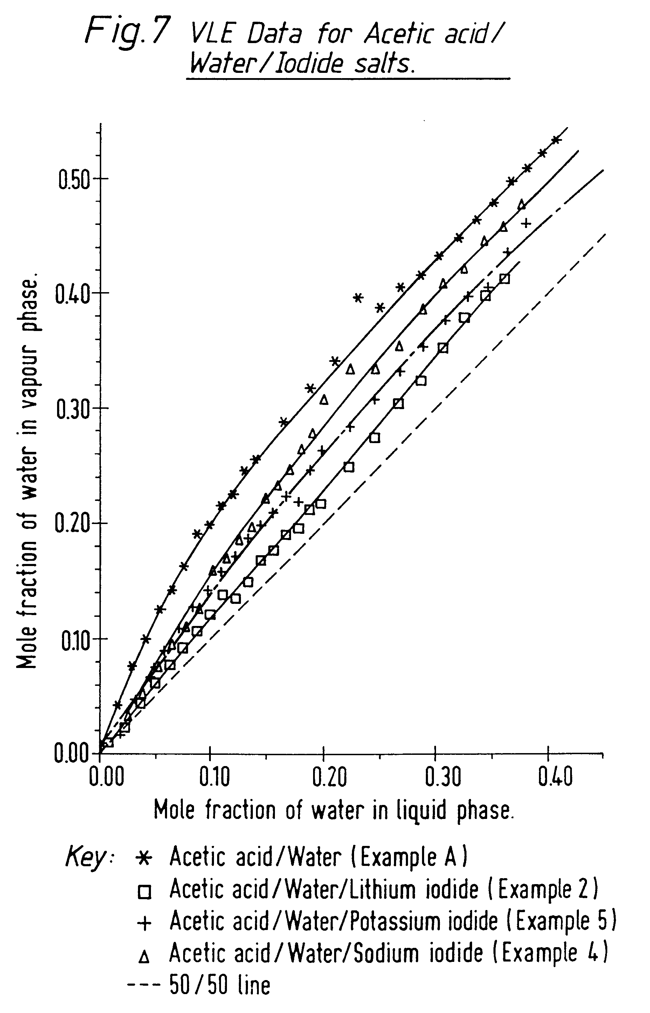

The results are shown in Figure 7 together with the results from Example 2 and Example A. These salts reduce the volatility of water relative to acetic acid but not to the same extent as for lithium iodide.

### Example 6

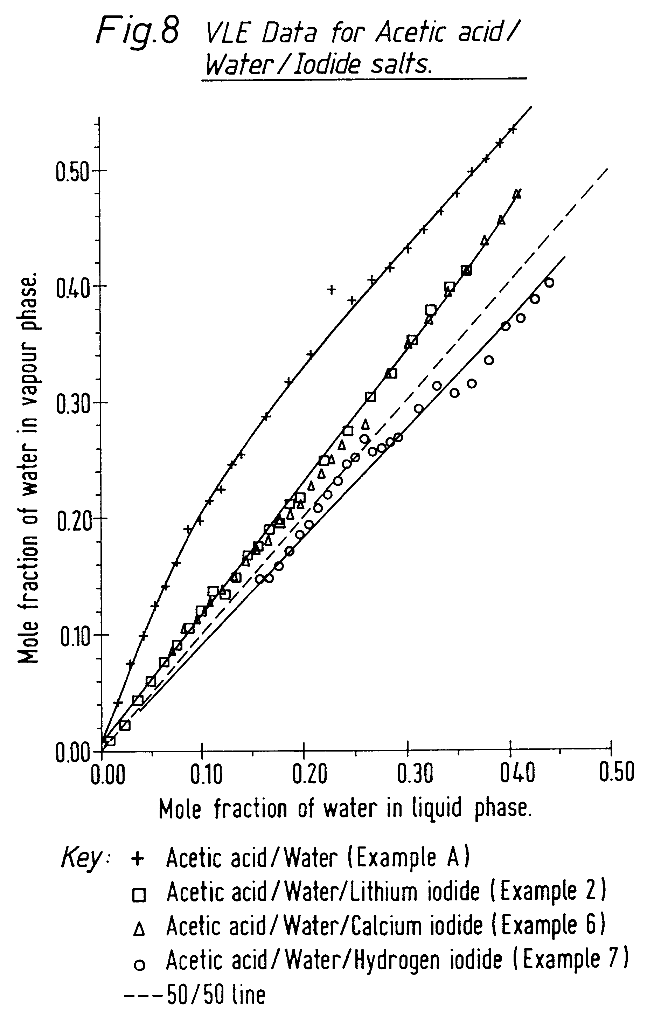

Example 2 was repeated using an equimolar amount of iodide (0.48 moles) added as calcium iodide tetrahydrate (17.30 weight %). Acetic anhydride (97.5g for 87.36g of iodide) was used to remove the water of crystallisation. The results are shown in Figure 8 together with the results for Examples A and 2. Calcium iodide shows an effect of similar magnitude to that of lithium iodide.

### Example 7

Example 6 was repeated using hydrogen iodide (0.24 moles) (53.11 g of 57% aqueous solution added to 446.89g of glacial acetic acid). The results shown in Figure 8 show the magnitude of the effect for hydrogen iodide to be greater than that for lithium iodide.

### Examples G, H and I (Comparative Examples)

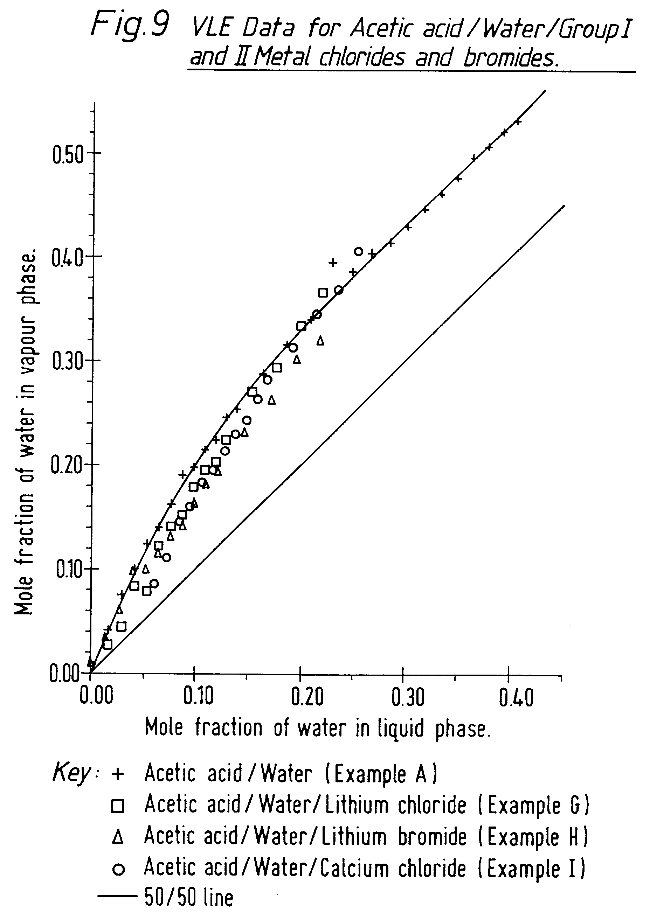

Example A was repeated using lithium chloride (4.00 weight %) lithium bromide (8.21 weight %) and calcium chloride (5.25 weight %) respectively and the results are shown in Figure 9. These salts do not reduce the volatility of the water component relative to acetic acid compared to the base case of acetic acid/water in Example A and are not according to the present invention.

### Example J (Comparative Example)

Example A was repeated using 4-ethyl-N-methyl pyridinium iodide (EMPI) (46.8 weight % in composition) as follows.

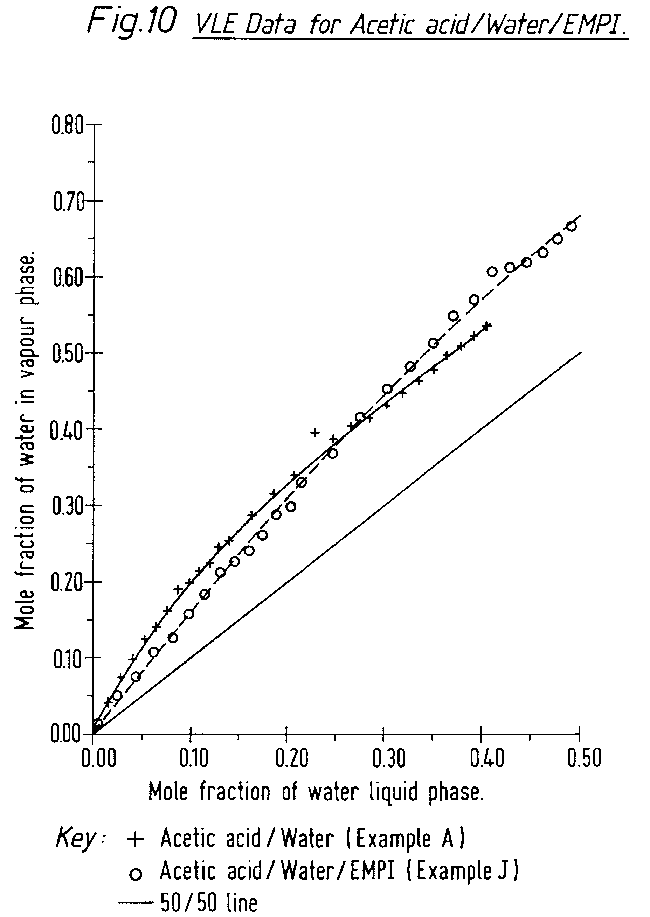

4-ethylpyridine (105.25g) was added to stirred glacial acetic acid (275.20g) over a period of about 15 minutes. Methyl iodide (134.18g) was added slowly over about 1 hour. The mixture was then refluxed for 2 days at the end of which analysis by ion selective electrode indicated 22% iodide and the reaction was assumed complete. This solution was used in the example. The results are shown in Figure 10 and do not indicate a reduction in water volatility relative to acetic acid compared to Example A.

### Example K (Comparative Example)

Example A was repeated using 4-ethylpyridinium iodide (EPI) (43.9 weight % in composition) prepared as follows. 4-ethylpyridine (101.12g) was added to stirred glacial acetic acid (188.3g) over a period of 15 minutes. Hydrogen iodide (210.5g of 57 weight % aqueous solution) was then added slowly over a period of 10 minutes. The solution was then refluxed for 2 hours at the end of which it was analysed for water and iodide content. This solution was used in the example. The results did not indicate a reduction in volatility of water relative to acetic acid over 15 to 25 weight % water.

## Claims

1. The use of one or more iodides of Group IA or Group IIA of the Periodic Table of the Elements or of hydrogen for suppressing the volatility of water relative to acetic acid in a process for the recovery of acetic acid from a composition comprising acetic acid and water, which process comprises partitioning the composition into a vapour phase comprising at least a portion of the acetic acid in the composition and a liquid phase comprising the remaining portion of the acetic acid in the composition and separating the phases.

2. The use as claimed in claim 1 in which the composition comprising acetic acid and water is subjected to elevated temperature and pressure and then introduced, together with or separately from the iodide, into a flash zone from which the vapour phase and liquid phase are withdrawn separately.

3. The use as claimed in claim 1 in which the composition comprising acetic acid and water is introduced into a fractional distillation zone comprising one or more separation stages, together with, or separately from, the iodide; lower boiling components of the composition are recovered as a head product stream from the distillation zone; higher boiling components of the composition are recovered as a base product stream and at least a portion of the acetic acid together with part of the water from the composition is recovered at a point above the base of the distillation zone.

4. The use as claimed in any one of the preceding claims in which the composition comprising acetic acid and water comprises the liquid reaction composition from a carbonylation process for the production of acetic acid.

5. The use as claimed in any one of the preceding claims in which the iodide comprises hydrogen iodide, calcium iodide,lithium iodide, potassium iodide and/or sodium iodide.

6. The use as claimed in any one of the preceding claims in which the iodide is formed in situ in the composition or liquid phase.

7. The use as claimed in any one of claims 1 to 5 in which the iodide is added to the composition prior to partitioning into vapour and liquid phases.

8. The use as claimed in any one of the preceding claims in which the composition is partitioned into vapour and liquid phases at a temperature in the range 50°C to 300°C.

9. The use as claimed in any one of the preceding claims in which the composition is partitioned into vapour and liquid phases at a pressure in the range 1 x 10⁴ to 5 x 10⁶ Pa (0.1 bara to 50 bara).

10. The use of one or more iodides of Group IA or Group IIA of the Periodic Table of the Elements or of hydrogen for suppressing the volatility of water relative to acetic acid in a process for the production of acetic acid which process comprises:
a) reacting methanol, methyl iodide, methyl acetate and/or dimethyl ether with carbon monoxide in the liquid phase in a reaction zone in the presence of a carbonylation catalyst and at least a finite amount of water at a temperature of 50 to 400°C and a carbon monoxide pressure of 7 x 10³ to 1 x 10⁸ Pag (1 to 15000 psig) to produce acetic acid,
b) withdrawing liquid reaction composition from the reaction zone,
c) introducing the liquid reaction composition into a flash zone at a pressure below that of the reaction zone with or without additional heating to produce a vapour phase comprising a portion of the acetic acid carbonylation product and water and a liquid phase,
d) introducing to the flash zone together with or separately from the liquid reaction composition, the one or more iodides of the elements of Group IA or Group IIA of the Periodic Table of the Elements or of hydrogen, and
e) separately removing from the flash zone the vapour phase and the liquid phase.

11. The use as claimed in claim 10 in which the carbonylation catalyst comprises a Group VIII metal compound and an alkyl iodide or bromide promoter.

12. The use as claimed in claims 10 or 11 in which the carbonylation catalyst additionally comprises a co-promoter.

13. The use as claimed in claim 12 in which the co-promoter is lithium iodide.

14. The use as claimed in any one of claims 10 to 13 in which the iodide comprises hydrogen iodide, calcium iodide, lithium iodide, potassium iodide and/or sodium iodide.

15. The use as claimed in any one of claims 1 to 10 in which the mole ratio of water:acetic acid in the composition comprising water and acetic acid is from 0.001:0.999 to 0.9:0.1.

16. The use as claimed in any one of claims 11 to 14 in which the mole ratio of water:acetic acid in the liquid reaction composition is from 0.001:0.999 to 0.9:0.1.

## Patentansprüche

1. Die Verwendung von einem oder mehreren Iodiden der Gruppe IA oder der Gruppe IIA des Periodischen Systems der Elemente oder von Wasserstoff zur Unterdrückung der Flüchtigkeit von Wasser bezüglich von Essigsäure in einem Verfahren für die Rückgewinnung von Essigsäure aus einer Zusammensetzung, enthaltend Essigsäure und Wasser, welches Verfahren das Unterteilen der Zusammensetzung in eine Dampfphase, enthaltend zumindest einen Teil der Essigsäure in der Zusammensetzung, und in eine Flüssigphase, enthaltend den restlichen Teil der Essigsäure in der Zusammensetzung, und das Trennen der Phasen, umfaßt.

2. Die Verwendung gemäß Anspruch 1, bei welcher die Essigsäure und Wasser enthaltende Zusammensetzung einer erhöhten Temperatur und Druck unterworfen wird und dann, zusammen mit oder getrennt von dem Iodid, in eine Entspannungszone eingeführt wird, aus welcher die Dampfphase und die Flüssigphase getrennt abgezogen werden.

3. Die Verwendung gemäß Anspruch 1, in welcher die Essigsäure und Wasser enthaltende Zusammensetzung in eine Fraktionierdestillationszone, enthaltend eine oder mehrere Trennstufen, eingeführt wird, zusammen mit, oder getrennt von, dem Iodid; niedrigersiedende Komponenten der Zusammensetzung als Kopfprodukt-Strom aus der Destillationszone gewonnen werden; höhersiedende Komponenten der Zusammensetzung als ein Bodenprodukt-Strom gewonnen werden, und zumindest ein Teil der Essigsäure zusammen mit einem Teil des Wassers aus der Zusammensetzung an einem Punkt oberhalb des Bodens der Destillationszone gewonnen wird.

4. Die Verwendung gemäß irgendeinem der vorstehenden Ansprüche, bei welcher die Essigsäure und Wasser enthaltende Zusammensetzung die Flüssigreaktion-Zusammensetzung aus einem Carbonylierungsverfahren für die Herstellung von Essigsäure enthält.

5. Die Verwendung gemäß irgendeinem der vorstehenden Ansprüche, bei welcher das Iodid Iodwasserstoff, Calciumiodid, Lithiumiodid, Kaliumiodid und/oder Natriumiodid umfaßt.

6. Die Verwendung gemaß irgendeinem der vorstehenden Ansprüche, bei welcher das Iodid in der Zusammensetzung oder der Flüssigphase in situ gebildet wird.

7. Die Verwendung gemäß irgendeinem der Ansprüche 1 bis 5, bei welcher das Iodid zu der Zusammensetzung vor der Unterteilung in Dampf- und Flüssigphase zugesetzt wird.

8. Die Verwendung gemäß irgendeinem der vorstehenden Ansprüche, bei welcher die Zusammensetzung in Dampf- und Flüssigphase bei einer Temperatur im Bereich von 50°C bis 300°C unterteilt wird.

9. Die Verwendung gemäß irgendeinem der vorstehenden Ansprüche, bei welcher die Zusammensetzung in Dampf- und Flüssigphase bei einem Druck im Bereich von 1 × 10⁴ bis 5 × 10⁶ Pa (0,1 bar bis 50 bar) unterteilt ist.

10. Die Verwendung von einem oder mehreren Iodiden der Gruppe IA oder Gruppe IIA des Periodischen Systems der Elemente oder von Wasserstoff zur Unterdrückung der Flüchtigkeit von Wasser bezüglich von Essigsäure in einem Verfahren für die Herstellung von Essigsäure, welches Verfahren umfaßt:
(a) Das Umsetzen von Methanol, Methyliodid, Methylacetat und/oder Dimethylether mit Kohlenmonoxid in der Flüssigphase in einer Reaktionszone in Gegenwart eines Carbonylierungskatalysators und bei zumindest einer begrenzten Menge an Wasser bei einer Temperatur von 50° bis 400°C und einem Kohlenmonoxid-Druck von 7 × 10³ bis 1 × 10⁸ Pag (1 bis 15 000 psig) zur Herstellung von Essigsäure,
(b) das Abziehen der flüssigen Reaktionszusammensetzung aus der Reaktionszone,
(c) das Einführen der flüssigen Reaktionszusammensetzung in eine Entspannungszone bei einem Druck unterhalb desjenigen der Reaktionszone mit oder ohne zusätzlichem Erhitzen zur Herstellung einer Dampfphase, enthaltend einen Teil des Essigsäure-Carbonylierungsprodukts und Wasser und eine Flüssigphase,
(d) das Einführen in die Entspannungszone, zusammen mit oder getrennt von der flüssigen Reaktionszusammensetzung, des einen oder von mehreren Iodiden der Elemente der Gruppe IA oder der Gruppe IIA des Periodischen Systems der Elemente, oder von Wasserstoff, und
(e) das getrennte Entfernen der Dampfphase und der Flüssigphase aus der Entspannungszone.

11. Die Verwendung gemäß Anspruch 10, bei welcher der Carbonylierungskatalysator eine Gruppe VIII-Metallverbindung und einen Alkyliodid- oder -bromid-Promotor enthält.

12. Die Verwendung gemäß Anspruch 10 oder 11, bei welche der Carbonylierungskatalysator zusätzlich einen Co-Promotor enthält.

13. Die Verwendung gemäß Anspruch 12, bei welcher der Co-Promotor Lithiumiodid ist.

14. Die Verwendung gemäß irgendeinem der Ansprüche 10 bis 13, bei welcher das Iodid Iodwasserstoff, Calciumiodid, Lithiumiodid, Kaliumiodid und/oder Natriumiodid umfaßt.

15. Die Verwendung gemäß irgendeinem der Ansprüche 1 bis 10, bei welcher das Molverhältnis von Wasser : Essigsäure in der Wasser und Essigsäure enthaltenden Zusammensetzung im Bereich von 0,001 : 0,999 bis 0,9 : 0,1 liegt.

16. Die Verwendung gemäß irgendeinem der Ansprüche 11 bis 14, bei welcher das Molverhältnis von Wasser : Essigsäure in der flüssigen Reaktionszusammensetzung im Bereich von 0,001 : 0,999 bis 0,9 : 0,1 liegt.

## Revendications

1. Utilisation d'un ou plusieurs iodures du Groupe IA ou du Groupe IIA du Tableau Périodique des Eléments ou d'iodure d'hydrogène pour supprimer la volatilité de l'eau par rapport à l'acide acétique dans un procédé pour séparer l'acide acétique d'une composition comprenant de l'acide acétique et de l'eau, procédé qui comprend le partage de la composition en une phase vapeur comprenant au moins une partie de l'acide acétique présent dans la composition et une phase liquide comprenant la partie restante de l'acide acétique présent dans la composition, et la séparation des phases.

2. Utilisation suivant la revendication 1, dans laquelle la composition comprenant de l'acide acétique et de l'eau est soumise à une température et une pression élevées et est ensuite introduite, conjointement avec ou séparément de l'iodure, dans une zone de détente instantanée de laquelle sont déchargées séparément la phase vapeur et la phase liquide.

3. Utilisation suivant la revendication 1, dans laquelle la composition comprenant de l'acide acétique et de l'eau est introduite dans une zone de distillation fractionnée comprenant un ou plusieurs étages de séparation, conjointement avec, ou séparément de, l'iodure ; les constituants à plus bas point d'ébullition de la composition sont recueillis sous forme d'un courant de produits de tête provenant de la zone de distillation ; les constituants à plus haut point d'ébullition de la composition sont recueillis sous forme d'un courant de produits de base et au moins une partie de l'acide acétique conjointement avec une partie de l'eau de la composition est recueillie à un point situé au-dessus de la base de la zone de distillation.

4. Utilisation suivant l'une quelconque des revendications précédentes, dans laquelle la composition comprenant de l'acide acétique et de l'eau consiste en la composition réactionnelle liquide provenant d'un procédé de carbonylation pour la production d'acide acétique.

5. Utilisation suivant l'une quelconque des revendications précédentes, dans laquelle l'iodure consiste en iodure d'hydrogène, iodure de calcium, iodure de lithium, iodure de potassium et/ou iodure de sodium.

6. Utilisation suivant l'une quelconque des revendications précédentes, dans laquelle l'iodure est formé in situ dans la composition ou phase liquide.

7. Utilisation suivant l'une quelconque des revendications 1 à 5, dans laquelle l'iodure est ajouté à la composition avant le partage en une phase vapeur et une phase liquide.

8. Utilisation suivant l'une quelconque des revendications précédentes, dans laquelle la composition est partagée en une phase vapeur et une phase liquide à une température comprise dans l'intervalle de 50°C à 300°C.

9. Utilisation suivant l'une quelconque des revendications précédentes, dans laquelle la composition est partagée une phase vapeur et une phase liquide sous une pression comprise dans l'intervalle de 1 x 10⁴ à 5 x 10⁶ Pa (0,1 bar à 50 bars en valeur absolue).

10. Utilisation d'un ou plusieurs iodures du Groupe IA ou du Groupe IIA du Tableau Périodique des Eléments ou d'iodure d'hydrogène pour supprimer la volatilité de l'eau par rapport à l'acide acétique dans un procédé de production d'acide acétique, procédé qui comprend :
a) la réaction de méthanol, d'iodure de méthyle, d'acétate de méthyle et/ou d'éther diméthylique avec le monoxyde de carbone en phase liquide dans une zone réactionnelle en présence d'un catalyseur de carbonylation et d'au moins une quantité finie d'eau à une température de 50 à 400°C et sous une pression de monoxyde de carbone de 7 x 10³ à 1 x 10⁸ Pa au manomètre (1 à 15 000 psig) pour produire de l'acide acétique,
b) le déchargement de la composition réactionnelle liquide de la zone de réaction,
c) l'introduction de la composition réactionnelle liquide dans une zone de détente instantanée à une pression inférieure à celle de la zone réactionnelle avec ou sans un chauffage supplémentaire pour produire une phase vapeur comprenant une partie du produit de carbonylation consistant en acide acétique et de l'eau et une phase liquide,
d) l'introduction dans la zone de détente instantanée, conjointement avec, ou séparément de, la composition réactionnelle liquide, du ou des iodures des éléments du Groupe IA ou du Groupe IIA du Tableau Périodique des Eléments ou de l'iodure d'hydrogène, et
e) l'évacuation, séparément, de la zone de détente instantanée de la phase vapeur et de la phase liquide.

11. Utilisation suivant la revendication 10, dans laquelle le catalyseur de carbonylation comprend un composé d'un métal du Groupe VIII et un promoteur consistant en un iodure ou bromure d'alkyle.

12. Utilisation suivant la revendication 10 ou 11, dans laquelle le catalyseur de carbonylation comprend en outre un copromoteur.

13. Utilisation suivant la revendication 12, dans laquelle le copromoteur est l'iodure de lithium.

14. Utilisation suivant l'une quelconque des revendications 10 à 13, dans laquelle l'iodure consiste en iodure d'hydrogène, iodure de calcium, iodure de lithium, iodure de potassium et/ou iodure de sodium.

15. Utilisation suivant l'une quelconque des revendications 1 à 10, dans laquelle le rapport molaire eau:acide acétique dans la composition comprenant de l'eau et de l'acide acétique est compris dans l'intervalle de 0,001:0,999 à 0,9:0,1.

16. Utilisation suivant l'une quelconque des revendications 11 à 14, dans laquelle le rapport molaire eau:acide acétique de la composition réactionnelle liquide est compris dans l'intervalle de 0,001:0,999 à 0,9:0,1.
